# EUROPEAN PATENT SPECIFICATION

(11) **EP 1 592 802 B1**
(45) Date of publication and mention of the grant of the patent: **28.03.2012**
(21) Application number: 04705979.5
(22) Date of filing: 28.01.2004
(51) Int. Cl.: C12Q 1/00, G01N 33/487, G01N 33/543

(54) **POLYMERIC MEMBRANES FOR USE IN ELECTROCHEMICAL SENSORS**
POLYMERMEMBRANEN ZUR VERWENDUNG IN ELEKTROCHEMISCHEN SENSOREN
MEMBRANES POLYMERES POUR CAPTEURS ELECTROCHIMIQUES

(30) Priority: 11.02.2003 US 364840
(43) Date of publication of application: 09.11.2005
(73) Proprietor: Instrumentation Laboratory Company, Lexington, MA 02142 (US)
(72) Inventor: COSOFRET, Vasile, V., Acton, MA 01720 (US)
(74) Representative: Kirkham, Nicholas Andrew
(86) International application number: PCT/US2004/002221
(87) International publication number: WO 2004/072606

(56) References cited:
- EP-A- 0 138 150
- EP-A- 0 654 664
- US-A- 4 973 394
- US-A- 5 200 051
- US-A1- 2003 000 833
- LINDNER E ET AL: "RESPONSES OF H+ SELECTIVE SOLVENT POLYMERIC MEMBRANE ELECTRODES FABRICATED FROM MODIFIED PVC MEMBRANES" TALANTA, ELMSFORD,NY, US, vol. 40, no. 7, 1993, pages 957-967, XP008003225
- COSOFRET V V ET AL: "Electroanalytical and biocompatibility studies on carboxylated poly(vinyl chloride) membranes for microfabricated array sensors." THE ANALYST. NOV 1994, vol. 119, no. 11, November 1994 (1994-11), pages 2283-2292, XP009035080 ISSN: 0003-2654
- J. L. F. C. LIMA & M. C. B. S. M. MONTENEGRO: "Dopamine Ion-Selective Electrode for Potentiometry in Pharmaceutical Preparations" MICROCHIMICA ACTA, vol. 131, no. 3-4, August 1999 (1999-08), pages 187-190, XP002291904 SPRINGER-VERLAG WIEN

## Description

### Technical Field

The present invention is related to the field of electrochemical sensors, particularly to the reduction of interference with the measurements of such sensors caused by contaminants in an analytical sample. -

### Background Information

Researchers and clinicians often need to measure the concentration of various analytes in biological samples. These analytes include dissolved gases (e.g. carbon dioxide), ions (e.g. hydrogen, sodium, potassium, calcium, lithium, ammonium, and magnesium), and biologically active molecules (e.g. urea). In many cases, the biological sample is a body fluid taken from a patient during an office visit or while undergoing surgery. Proper diagnosis and treatment often depend upon the accuracy of these measurements and the speed with which they are obtained.

An electrochemical sensor system is an analytical tool that can be used to measure the concentration of an analyte in a biological sample. The electrochemical sensor contains a physical transducer, such as a metal electrode, separated from the analytical sample by at least one semi-permeable membrane. The membrane imparts the selectivity to a given electrochemical sensor. The proper choice of membrane components allows for electrochemical sensors that can accurately detect and measure analytes in complex mixtures, such as whole blood.

Certain contaminants that are often present in analytical samples can compromise the accuracy of the electrochemical sensor by diffusing through the membrane and interfering with the metal electrode. In the case of ion-selective electrodes (ISEs), the contaminant can be a drug species, such as the anesthetic thiopental sodium (thiopental), for example. The result of such contamination on the ISE can range from mild interference to one of clinical significance, depending on the concentration of the contaminant in the sample.
LINDNER et al., Talanta 40/7, 1993, pp. 957 - 967, discloses the responses of H(+) selective solvent polymeric membrane electrodes fabricated from modified PVC membranes.
COSOFRET et al., Analyst 119, 1994, pp. 2283 - 2292 describes electroanalytical and biocompatibility studies on carboxylated poly(vinyl chloride) membranes for microfabricated sensors.
LIMA et al., Microchimica Acta 131, 1999, pp. 187 -190, discloses a dopamine ion-selective electrode for potentiometry in pharmaceutical preparations.

### Summary of the Invention

The present invention provides a method for reducing the interference caused by contaminants on electrochemical sensors used for the detection and/or measurement of an analyte in a biological sample. The electrochemical sensor according to the present invention does not exhibit large negative shifts in potential after assaying an analytical sample contaminated with, for example, a lipophilic anionic drug species. In addition, an electrochemical sensor according to the present invention exhibits a reduction in bulk membrane resistance, which is important for sensor stability and the reproducibility of sensor measurements. An electrochemical sensor fabricated according to the present invention displays a stable baseline that is quickly recovered after assaying a sample, which results in shorter measurement times. These shorter measurement times can lead to increased throughput for an analytical instrument that incorporates such an electrochemical sensor.

In general, in one aspect, the present invention features an in vitro diagnostic method, comprising: selecting patients treated with lipophilic anionic agents selected from the group consisting of an analgesic, an anesthetic, thiopental sodium, phenytoin, ibuprofen, salicylate, valproate and eamino-caproate; providing a sensor that reduces interference caused by lipophilic anionic agents comprising an electrode and a carboxylated polyvinyl chloride polymeric membrane;
contacting a body fluid from said patient with said sensor;
measuring a target analyte in said patient body fluid; and,
determining a diagnosis from the measurement of said target analyte.
The PVC-COOH based polymeric membrane reduces the interference with the detection and/or measurement of the analyte in the biological sample caused by the contaminants

Embodiments of this aspect of the invention may include the following features. The contaminant that the PVC-COOH based polymeric membrane prevents from interfering with the electrode may comprise a lipophilic anionic species. The contaminant may be an anesthetic or an analgesic. More specifically, the contaminant may be thiopental sodium (thiopental), or it may be phenytoin, ibuprofen, fenoprofen, salicylate, valproate, or ε-amino-caproate. The analyte measured and/or detected by the electrode may be an inorganic cation. The analyte may also be a dissolved gas, or it may be a biological metabolite, such as a carbohydrate, peptide, lipid, nucleotide, or urea. The biological sample itself may be a body fluid, such as blood or urine. The PVC-COOH that is included in the polymeric membrane may contain 0.1 to 5 % carboxyl groups by weight.

The foregoing and other objects, aspects, features, and advantages of the invention will become more apparent from the following description and from the claims.

### Brief Description of the Drawings

In the drawings, like reference characters generally refer to the same parts throughout the different views. Also, the drawings are not necessarily to scale, emphasis instead generally being placed upon illustrating the principles of the invention.

Fig. 1 is a schematic diagram of the components of an embodiment of an electrochemical sensor system according to the invention, including a sensor cartridge with an electrode card and sample inlet, a peristaltic pump, and a microprocessor.

Fig. 2 illustrates a frontal view of an embodiment of an electrode card according to the invention.

Fig. 3 illustrates a cross sectional view of an embodiment of an ion-selective electrode (ISE) according to the invention.

Fig. 4 illustrates a cross sectional view of an embodiment of a carbon dioxide (CO₂) electrode according to the invention.

Fig. 5 illustrates a cross sectional view of an embodiment of an enzyme electrode according to the invention.

Fig. 6 is a table containing examples of polymeric membrane components and their respective weight percentages for four different ISEs.

Fig. 7 is a graphical representation of the chronopotentiometric responses of an electrode card that includes a sodium ISE with a polymeric membrane containing high molecular weight polyvinyl chloride (HMW-PVC), with measurements taken before and after assaying a sodium-containing sample contaminated with 10 mg/dL thiopental sodium (thiopental).

Fig. 8 is a graphical representation of the chronopotentiometric responses of an electrode card that includes five sodium ISEs with polymeric membranes containing carboxylated polyvinyl chloride (PVC-COOH) according to the invention, with measurements taken before and after assaying a sodium-containing sample contaminated with 10 mg/dL thiopental.

Fig. 9 is a graphical representation of the chronopotentiometric responses of an electrode card that includes a potassium ISE with a polymeric membrane containing HMW-PVC, with measurements taken before and after assaying a potassium-containing sample contaminated with 10 mg/dL thiopental.

Fig. 10 is a graphical representation of the chronopotentiometric responses of an electrode card that includes five potassium ISEs with polymeric membranes containing PVC-COOH according to the invention, with measurements taken before and after assaying a potassium-containing sample contaminated with 10 mg/dL thiopental.

Fig. 11 is a graphical representation of the chronopotentiometric responses of an electrode card that includes a calcium ISE with a polymeric membrane containing HMW-PVC, with measurements taken before and after assaying a calcium-containing sample contaminated with 10 mg/dL thiopental.

Fig. 12 is a graphical representation of the chronopotentiometric responses of an electrode card that includes five calcium ISEs with polymeric membranes containing PVC-COOH according to the invention, with measurements taken before and after assaying a calcium-containing sample contaminated with 10 mg/dL thiopental.

Fig. 13 is a graphical representation of the chronopotentiometric responses of an electrode card that includes a pH electrode with a polymeric membrane containing HMW-PVC, with measurements taken before and after assaying a pH buffered sample contaminated with 10 mg/dL thiopental.

Fig. 14 is a graphical representation of one of the chronopotentiometric responses of an electrode card that includes five pH electrodes with polymeric membranes containing PVC-COOH according to the invention, with measurements taken before and after assaying a pH buffered sample contaminated with 10 mg/dL thiopental.

Fig. 15 is a graphical representation of the chronopotentiometric responses of an electrode card that includes a CO₂ electrode with a polymeric membrane containing HMW-PVC, with measurements taken before and after assaying a sample contaminated with 10 mg/dL thiopental.

Fig. 16 is a graphical representation of the chronopotentiometric responses of an electrode card that includes four CO₂ electrodes with polymeric membranes containing PVC-COOH according to the invention, with measurements taken before and after assaying a sample contaminated with 10 mg/dL thiopental.

Fig. 17 is a graphical representation of the bulk membrane resistance for an ISE with a polymeric membrane containing HMW-PVC.

Fig. 18 is a graphical representation of the bulk membrane resistance for an ISE with a polymeric membrane containing PVC-COOH.

Fig 19 is a graphical representation comparing sodium concentration values in whole blood samples determined by a sodium ISE with a polymeric membrane containing HMW-PVC against those determined by an electrode card including five sodium ISEs with polymeric membranes containing PVC-COOH according to the invention.

Fig. 20 is a graphical representation comparing potassium concentration values in whole blood samples determined by a potassium ISE with a polymeric membrane containing HMW-PVC against those determined by an electrode card including five potassium ISEs with polymeric membranes containing PVC-COOH according to the invention.

Fig. 21 is a graphical representation of the negative drift in potential over time of a sodium ISE with a polymeric membrane containing PVC-COOH according to the invention.

### Description

The present invention provides a method for reducing the interference caused by contaminants on electrochemical sensors used for the detection and/or measurement of an analyte in a biological sample. Specifically, the invention describes an electrochemical sensor having carboxylated polyvinyl chloride (PVC-COOH) as a polymer component of its polymeric membrane. The electrochemical sensor according to the invention can be configured to detect several analytes, including dissolved gases (e.g. carbon dioxide), ions (e.g. hydrogen, sodium, potassium, calcium, lithium, ammonium, and magnesium), and biological metabolites (e.g. urea).

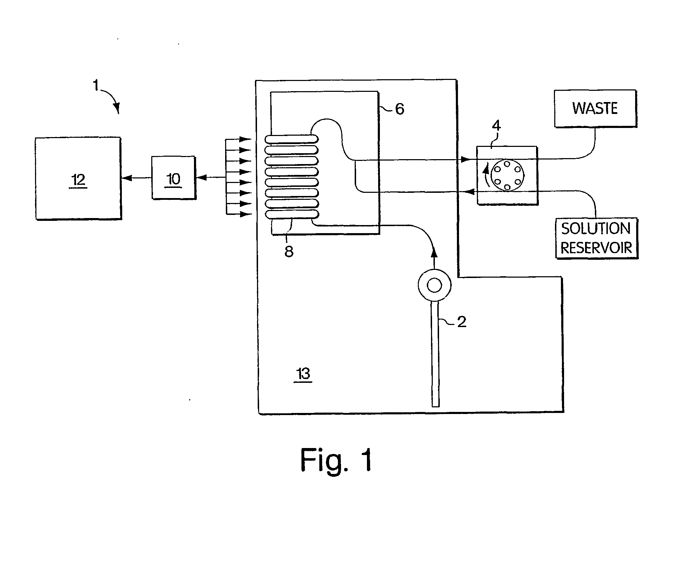

An electrochemical sensor according to the invention can be incorporated into an electrochemical sensor system. Referring to Figure 1, in one embodiment according to the invention, the electrochemical sensor system 1 has an inlet 2 where the biological sample is introduced into the electrochemical sensor system 1. A peristaltic pump 4 moves a sample, such as a body fluid sample, through the inlet 2 and into an electrode card 6. The electrode card 6 contains one or more electrodes 8 that detect and measure analytes of interest in the sample. An electrical interface 10 connects the electrode card 6 to a microprocessor 12. Signals from the electrode card 6 pass to the microprocessor 12 to allow for storage and display of the signals. Signals from the microprocessor 12 pass to the electrode card 6 to allow for control over measurement conditions, such as the polarization voltage of an electrode. In one embodiment according to the invention, the sample inlet 2 and the electrode card 6 are contained within a disposable cartridge 13, which can be detached from the remaining elements of the electrochemical sensor system 1 and replaced after use.

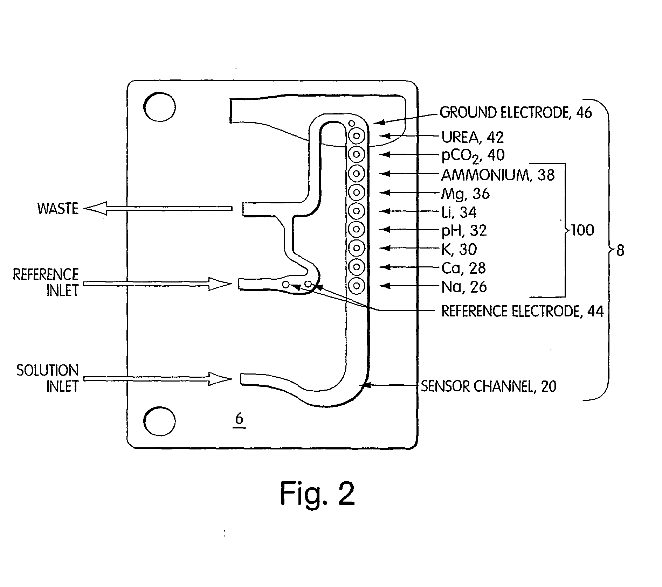

Referring to Figure 2, in one embodiment according to the invention, the electrode card 6 includes a rigid, substantially rectangular card made of polyvinyl chloride (PVC). A channel 20 is located within the electrode card 6, through which a biological sample or a reference solution can flow. One or more electrodes 8 can be embedded within the channel 20. When a sample is passed through the electrode card 6, it flows through the channel 20 and over the electrodes 8, allowing for detection and/or measurement of the analyte(s) of interest.

Referring to Figure 2, the electrodes 8 that can be incorporated into the electrode card 6 include ion-selective electrodes (ISEs) 100, electrodes for analyzing dissolved gases (gas electrodes), and electrodes which use an enzyme-based detection system (enzyme electrodes). For example, the electrodes may detect sodium 26, calcium 28, potassium 30, pH 32, lithium 34, magnesium 36, ammonium 38, carbon dioxide 40, and urea 42.

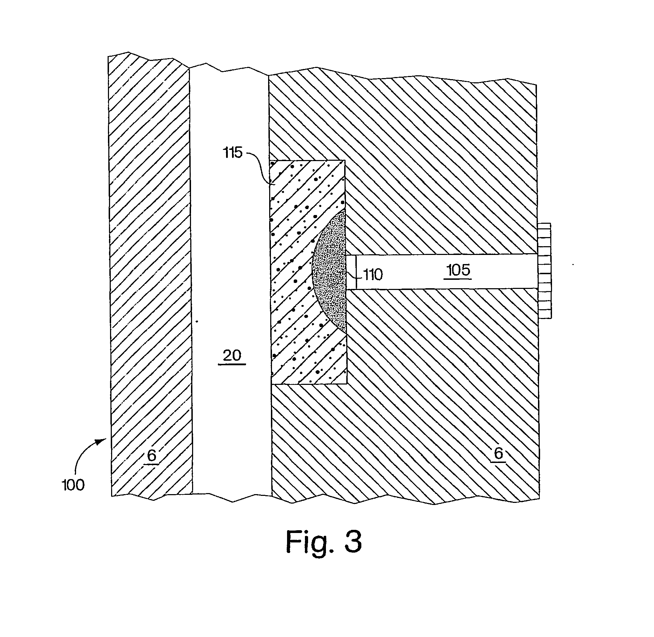

Referring to Figure 3, in one embodiment according to the invention, an ISE 100 comprises a metal element 105, an inner solution layer 110, and a polymeric membrane 115. The metal element 105 is embedded in the PVC of an electrode card 6, and the inner solution layer 110 covers the exposed end of the metal element 105. The inner solution layer 110 may contain, for example, 2-[N-morpholino]ethanesulfonic acid (MES) buffer. The polymeric membrane 115 is an ion-selective membrane that separates the inner solution layer 110 from an analytical sample (for example, a body fluid sample) that passes through the channel 20 in the electrode card 6. The composition of the polymeric membrane 115 determines the selectivity of the ISE 100 for a particular ion. In a particular embodiment according to the invention, PVC-COOH is a component of the polymeric membrane 115.

Referring back to Figure 2, to measure the concentration of an ion in an analytical sample, an ISE 100 must work in tandem with a reference electrode 44. If the ion that the ISE 100 is designed to detect is present in the analytical sample, an electrical potential is generated across the polymeric membrane 115 that depends on the difference between the concentration of the analyte in the inner solution layer 110, illustrated in Figure 3, and its concentration in the analytical sample. The difference in electrical potential between the ISE 100 and the reference electrode 44 is directly proportional to the change in the logarithm of the concentration of the measured ion in the analytical sample.

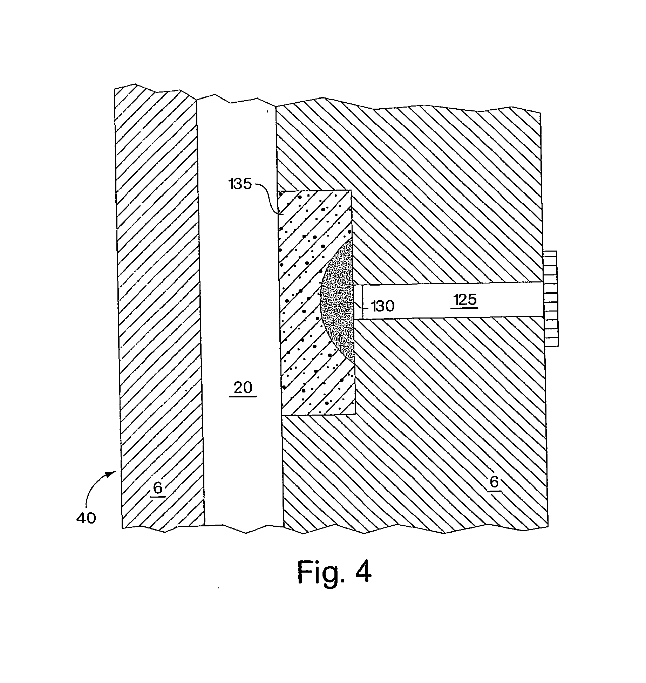

Referring to Figure 4, in one embodiment according to the invention, a carbon dioxide (CO₂) electrode 40, one type of gas electrode, comprises a metal element 125, an inner solution layer 130, and a polymeric membrane 135. The CO₂ electrode 40 is functionally similar to an ISE 100, except that the inner solution layer 130 of the CO₂ electrode 40 is bicarbonate buffer. Referring to Figure 2, unlike an ISE 100, the CO₂ electrode 40 must work in tandem with a pH electrode 32.

Referring again to Figure 4, when CO₂ permeates the polymeric membrane 135 of the CO₂ electrode 40, it dissolves in the bicarbonate buffer of the inner solution layer 130 and changes the buffer pH, which changes the electrical potential of the CO₂ electrode 40. The inner solution layer of the pH electrode 32, however, is not affected by CO₂ in the analytical sample, so the pH electrode's potential remains constant. The difference in electrical potential between the CO₂ electrode 40 and the pH electrode 32 is proportional to the concentration of CO₂ in the sample. In one embodiment according to the invention, PVC-COOH can be a constituent of the polymeric membrane 135 of a CO₂ electrode 40.

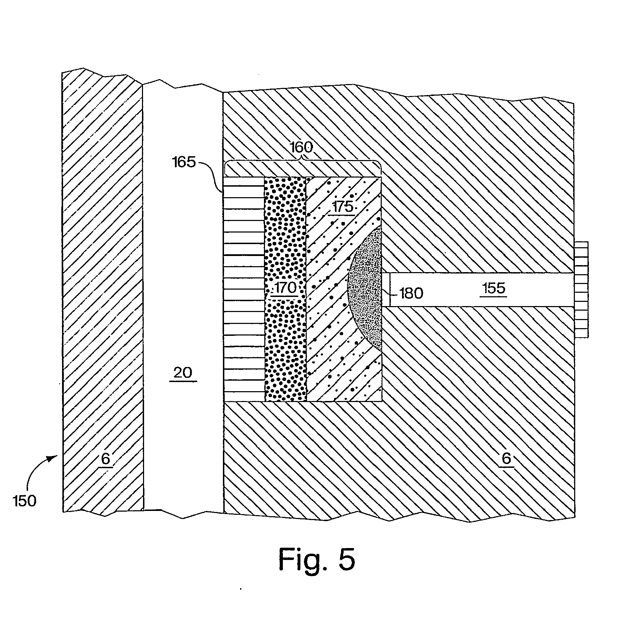

Figure 5 illustrates another embodiment according to the invention, an enzyme electrode 150 for detecting the presence and concentration of biological metabolites (such as a carbohydrate, peptide, lipid, nucleotide, or urea, for example) in an analytical sample. The enzyme electrode 150 comprises a metal element 155 embedded in an electrode card 6 and a composite membrane 160, which is located between the metal element 155 and an analytical sample flowing through a channel 20 in the electrode card 6. The composite membrane 160 includes an outer diffusional membrane 165 adjacent to the channel 20, an enzymatic layer 170, an ion-selective polymeric membrane 175, and an inner solution layer 180 adjacent to the metal element 155. The outer diffusional membrane 165 controls the diffusion of the analyte into the enzyme layer 170 and protects the other components of the enzyme electrode 150 from direct contact with the analytical sample in the channel 20. The enzyme layer 170 may include at least one enzyme, or a mixture of several enzymes, proteins, and stabilizers, that reacts with a particular analyte. If the analyte diffuses through the outer diffusional membrane 165, it can react with the enzyme(s) in enzyme layer 170 to produce a chemical byproduct, which can migrate through the ion-selective polymeric membrane 175. In the case of a urea sensor, the chemical byproduct can be ammonium ions, for example. An electrical potential is generated across the composite membrane 160 that depends on the concentration of the chemical byproduct, which is proportional to the concentration of the analyte of interest in the analytical sample. In one embodiment according to the invention, PVC-COOH is a constituent of the ion-selective polymeric membrane 175.

Referring again to Figure 3, the polymeric membrane of an ISE regulates the selectivity of the ISE 100 toward an analyte of interest. The polymeric membrane 115 includes at least four elements: a polymer, a plasticizer, an ionophore, and a lipophilic salt additive.

According to the invention, PVC-COOH is a polymer component of the polymeric membrane 115. PVC-COOH is polyvinyl chloride (PVC) polymer that has a percentage of its chlorine atoms replaced by carboxyl groups (COOH). In an embodiment accoding to the invention, the PVC-COOH can contain between 0.1 and 5 % COOH by weight, and in a particular embodiment of the invention, the PVC-COOH contains 1.8 % COOH by weight. The PVC-COOH prevents lipophilic anionic species, (such as analgesics and anesthetics, for example) in the analytical sample from permeating the polymeric membrane 115 and interfering with the ISE 100. Such lipophilic anionic species can include thiopental sodium (thiopental), phenytoin, ibuprofen, fenoprofen, salicylate, valproate, and ε-amino-caproate, for example. PVC-COOH can be mixed with another polymer (such as HMW-PVC or polyurethane, for example) to form the polymer component of the polymeric membrane. In a particular embodiment of the invention, PVC-COOH is not mixed with another polymer and is the only polymer component of the polymeric membrane.

The polymeric membrane 115 that includes PVC-COOH also exhibits enhanced adhesive properties to solid platforms, which is important for the long life and potential stability of an electrochemical sensor. In addition, an electrochemical sensor employing PVC-COOH in its polymeric membrane 115 shows better potential stability and reproducibility of sensor measurements due to a significant reduction in the membrane resistance conferred by the relatively polar polymeric membrane 115, as illustrated by Example 6 below. The precision and accuracy of a PVC-COOH sensor is comparable to known ISEs, including sensors based on high molecular weight polyvinyl chloride (HMW-PVC), as illustrated by Example 7 below.

The plasticizer component of the polymeric membrane 115 provides ion mobility within the membrane that is necessary to obtain effective ion transfer. The plasticizer must be compatible with the polymer component and must be a solvent for the ionophore. The plasticizer must also be sufficiently insoluble in water so that it does not migrate significantly into an aqueous sample in contact with the surface of the polymeric membrane 115. It is also desirable that the plasticizer be substantially non-volatile to extend the shelf-life of the electrode. Useful plasticizers include bis(2-ethylhexyl) sebacate (DOS) and o-nitrophenyl octyl ether (NPOE).

The ionophore used in the polymeric membrane 115 is capable of selectively associating with a specific ion. This feature of the ionophore is responsible for the ion-selectivity of an ISE. Examples of suitable ionophores for a sodium ISE include methyl monensin ester, calixarene derivatives, and other sodium-sensitive compounds. A monocyclic antibiotic (such as valinomycin, for example) can be used as an ionophore for a potassium ISE. An ionophore for a calcium ISE may be, for example, (-)-(R,R)-N,N'-(Bis(11-ethoxycarbonyl)undecyl)-N,N'-4,5-tetramethyl-3,6-dioxaoctanediamide; Diethyl N,N'-[(4R,5R)-4,5-dimethyl-1,8-dioxo-3,6-dioxaoctamethylene]-bis(12-methylaminododecanoate) (ETH 1001). An example of a suitable ionophore for a pH electrode and/or a carbon dioxide electrode is tridodecylamine (TDDA).

The lipophilic salt additive used in the polymeric membrane 115 serves to reduce membrane resistance and to reduce anion interference. Useful lipophilic salt additives include, for example, potassium tetrakis(4-chlorophenyl)borate (KTpCIPB) and potassium tetrakis[3,5-bis(trifluoromethyl)phenyl]borate (KTTFPB). The lipophilic salt additive, however, is not always essential to the function of the polymeric membrane 115 and can be omitted when certain analytes are targeted.

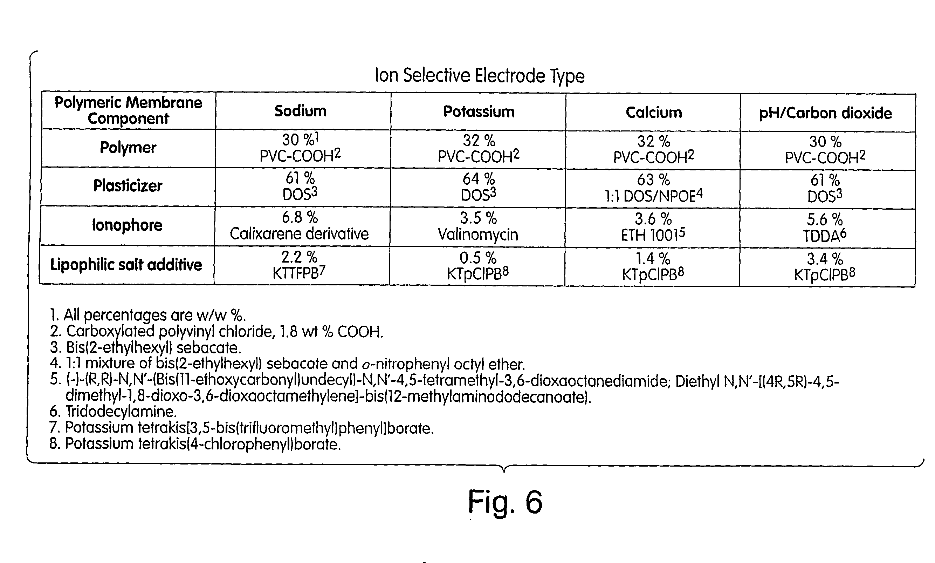

The efficiency of the polymeric membrane 115 in rejecting lipophilic anion drug contaminants is enhanced when the polymeric membrane 115 composition is optimized in terms of PVG-COOH/plasticizer ratio and by a proper selection of the type and ratios of ionophore and lipophilic salt additive. For example, the polymeric membrane 115 for a sodium ISE may contain 25-35 % PVC-COOH, 60-70 % DOS, 2-8 % calixarene derivative, and 1-3 % KTTFPB by weight. The polymeric membrane 115 for a potassium ISE may contain, for example, 25-35 % PVC-COOH, 60-70 % DOS, 1-5 % valinomycin, and 0-1 % KTpClPB by weight. The polymeric membrane 115 for a calcium ISE may contain, for example, 25-35 % PVC-COOH, 60-70 % 1:1 DOS/NPOE, 1-5 % ETH 1001, and 0.2-2 % KTpClPB by weight. The polymeric membrane 115 for a pH or CO₂ ISE may contain, for example, 25-35 % PVC-COOH, 60-70 % DOS, 2-7 % TDDA, and 1-4 % KTpCIPB by weight. Figure 6 illustrates examples of particular embodiments of suitable polymeric membrane 115 components and their respective weight ratios for a variety of electrochemical sensors.

Referring still to Figure 3, a polymeric membrane 115 according to the present invention can be formed by dissolving the appropriate amounts of polymer, plasticizer, ionophore, and lipophilic salt additive in a solvent, typically tetrahydrofuran (THF) or cyclohexanone, and applying this solution to the exposed surface of a metal element 105 embedded in an electrode card 6. For example, a potassium ISE can be fabricated by mixing PVC-COOH (1.8 wt % COOH), DOS, valinomycin and KTpClPB according to the ratios listed in Figure 6 to make a total mass of 630-650 mg. The mixture is dissolved in 3-3.5 mL THF, and 0.75 µL of the solution is applied to the exposed end of the metal element 105 (for example, a chloridized silver wire) embedded in the electrode card 6. Once the solvent evaporates, the same volume of membrane solution is applied two additional times with adequate drying time in between each application. Once the solvent has evaporated from the last application, the polymeric membrane 115 is formed and is bonded to the electrode card 6.

An ISE according to the present invention measures changes in the electric potential, measured in millivolts (mV), of an analytical sample that are due to changes in the concentrations of analytes within the sample. Similarly, a gas electrode according to the present invention measures changes in the electric potential of an analytical sample that are due to changes in the partial pressure of the gas dissolved in the sample. As practitioners skilled in the art are aware, electric potential values are related to concentration or partial pressure values according to the Nernst equation. In a particular embodiment according to the invention, software may be included in the electrochemical sensor system to convert electrical potential values measured by the electrode to concentration or partial pressure values of the measured analyte by using the Nernst equation.

In another aspect, the invention is a method for detecting the presence and/or measuring the concentration of an analyte in a body fluid (such as blood, for example) in the presence of a lipophilic anionic species (such as thiopental, for example) without interference by the lipophilic anionic species in detecting and/or measuring the analyte. The method of the invention provides an electrochemical sensor for detecting and/or measuring an analyte of interest in a body fluid that includes PVC-COOH as a polymer component of the sensor's polymeric membrane. A body fluid sample containing the analyte of interest and a lipophilic anionic contaminant is placed in contact with the electrochemical sensor that includes PVC-COOH as a polymer component of the sensor's polymeric membrane. The analyte of interest in the body fluid sample is then measured and/or detected by the electrochemical sensor with reduced interference by the lipophilic anionic contaminant.

The following examples are intended to illustrate, but not limit, the invention.

### Example 1

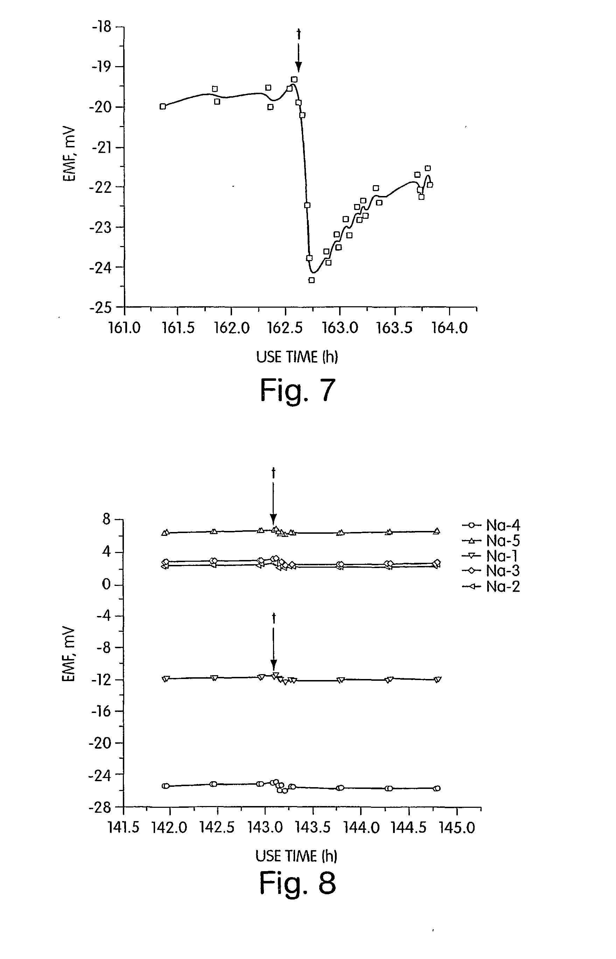

Figure 7 is a graphical representation of the chronopotentiometric responses recorded from an electrode card including a sodium ISE with a polymeric membrane containing HMW-PVC. An analytical sample containing a known concentration of sodium was introduced to the electrode card, and the ISE measured the concentration of sodium to be 142 mM. At time t, the analytical sample was changed to a solution containing the same concentration of sodium plus 10 mg/dL thiopental, and the ISE returned a sodium concentration value of 137 mM.

Figure 8 is a graphical representation of the chronopotentiometric responses recorded from an electrode card including five sodium ISEs with polymeric membranes containing PVC-COOH according to the invention. An analytical sample containing a known concentration of sodium was introduced to the electrode card, and all five ISEs measured the concentration of sodium to be 139 mM. At time t, the analytical sample was changed to a solution containing the same concentration of sodium plus 10 mg/dL thiopental, and all five electrodes returned sodium concentration values of 139 mM. The PVC-COOH based sodium ISEs did not exhibit the drift in potential displayed by the HMW-PVC based sodium ISE after analyzing a sample contaminated with thiopental, which illustrates the efficacy of PVC-COOH in preventing interference with sodium measurements caused by lipophilic anionic contaminants.

### Example 2

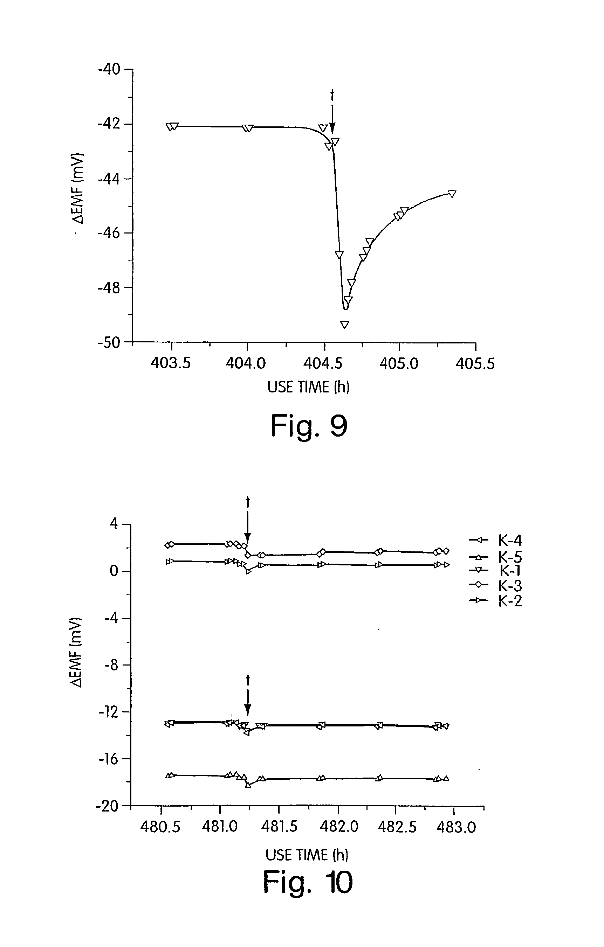

Figure 9 is a graphical representation of the chronopotentiometric responses recorded from an electrode card including a potassium ISE with a polymeric membrane containing HMW-PVC. An analytical sample containing a know concentration of potassium was introduced to the electrode card, and the ISE measured the concentration of potassium to be 3.2 mM. At time t, the analytical sample was changed to a solution containing the same concentration of potassium plus 10 mg/dL thiopental, and the ISE returned a potassium concentration value of 2.8 mM.

Figure 10 is a graphical representation of the chronopotentiometric responses recorded from an electrode card including five potassium ISEs with polymeric membranes containing PVC-COOH according to the invention. An analytical sample containing a known concentration of potassium was introduced to the electrode card, and all five ISEs measured the concentration of potassium to be 3.3 mM. At time t, the analytical sample was changed to a solution containing the same concentration of potassium plus 10 mg/dL thiopental, and all five electrodes returned potassium concentration values of 3.3 mM. The PVC-COOH based potassium ISEs exhibited negligible potential drifts after analyzing a sample contaminated with thiopental as compared to the drift displayed by the HMVV-PVC based potassium ISE, which illustrates the efficacy of PVC-COOH in preventing interference with potassium measurements caused by lipophilic anionic contaminants.

### Example 3

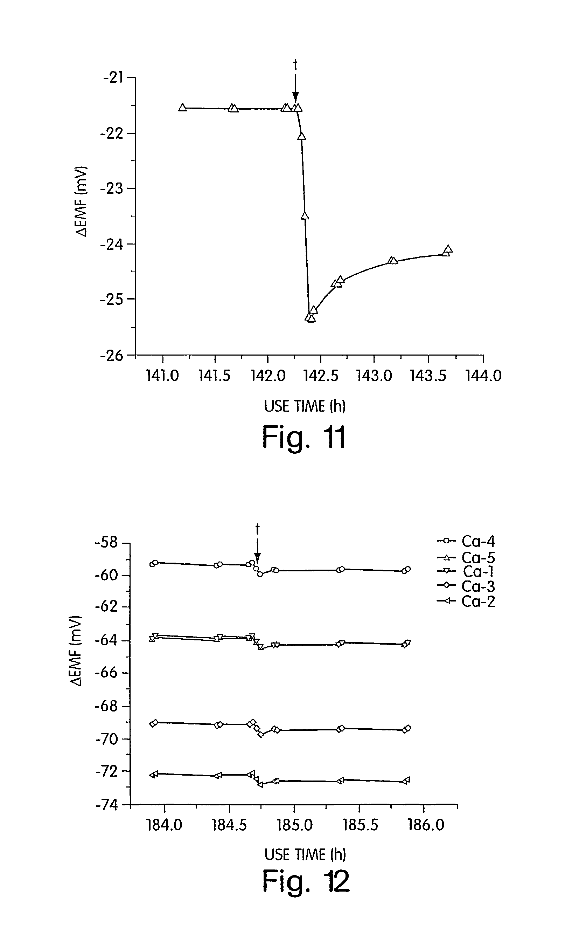

Figure 11 is a graphical representation of the chronopotentiometric responses recorded from an electrode card including a calcium ISE with polymeric membrane containing HMW-PVC. An analytical sample containing a known concentration of calcium was introduced to the electrode card, and the ISE measured the concentration of calcium to be 0.93 mM. At time t, the analytical sample was changed to a solution containing the same concentration of calcium plus 10 mg/dL thiopental, and the ISE returned a calcium concentration value of 0.81 mM.

Figure 12 is a graphical representation of the chronopotentiometric responses recorded from an electrode card including five calcium ISEs with polymeric membranes containing PVC-COOH according to the invention. An analytical sample containing a known concentration of calcium was introduced to the electrode card, and all five ISEs measured the concentration of calcium to be 0.87 mM. At time t, the analytical sample was changed to a solution containing the same concentration of calcium plus 10 mg/dL thiopental, and the five electrodes returned calcium concentration values of 0.85, 0.85, 0.86, 0.86, and 0.85 mM. The PVC-COOH based calcium ISEs did not exhibit as great a drift in potential as displayed by the HMW-PVC based calcium ISE after analyzing a sample contaminated with thiopental, which illustrates the efficacy of PVC-COOH in preventing interference with calcium measurements caused by lipophilic anionic contaminants.

### Example 4

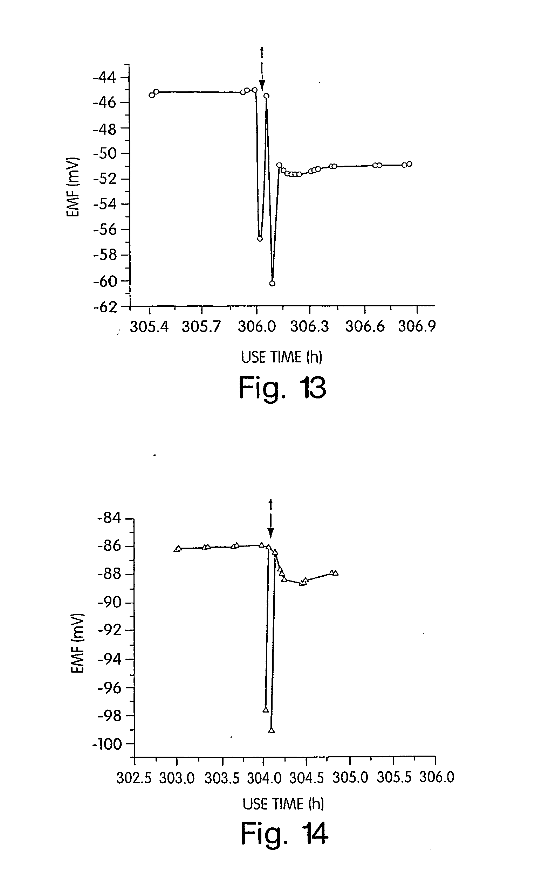

Figure 13 is a graphical representation of the chronopotentiometric responses recorded from an electrode card including a pH electrode with a polymeric membrane containing HMW-PVC. An analytical sample containing a buffer solution of known pH was introduced to the electrode card, and the electrode measured the pH to be 7.63. At time t, the analytical sample was changed to a solution containing the same buffer solution plus 10 mg/dL thiopental, and the electrode returned a pH value of 7.68.

Figure 14 is a graphical representation of one of the chronopotentiometric responses recorded from an electrode card including five pH electrodes with polymeric membranes containing PVC-COOH according to the invention. An analytical sample containing a buffer solution of known pH was introduced to the electrode card, and all five electrodes measured the pH to be 7.66. At time t, the analytical sample was changed to a solution containing the same buffer solution plus 10 mg/dL thiopental, and the five electrodes returned pH values of 7.68. The PVC-COOH based pH electrodes did not exhibit as great a drift in potential as displayed by the HMW-PVC based pH electrode after analyzing a sample contaminated with thiopental, which illustrates the efficacy of PVC-COOH in preventing interference with pH measurements caused by lipophilic anionic contaminants.

### Example 5

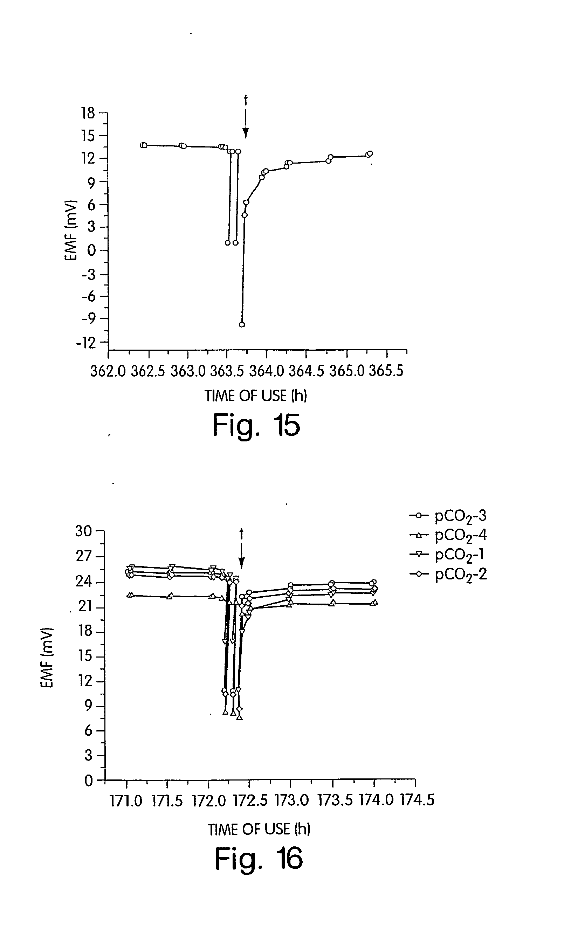

Figure 15 is a graphical representation of the chronopotentiometric responses recorded from an electrode card including a CO₂ electrode with polymeric membrane containing HMW-PVC. An analytical sample containing a buffer solution was introduced to the electrode card. Two measurements of the partial pressure of CO₂ in the solution were taken, and the results were averaged to yield a value of 66 mm Hg. At time t, the analytical sample was changed to a solution containing the same buffer solution plus 10 mg/dL thiopental, and the electrode returned CO₂ partial pressure value of 115 mm Hg.

Figure 16 is a graphical representation of the chronopotentiometric responses recorded from an electrode card including four CO₂ electrodes with polymeric membranes containing PVC-COOH according to the invention. An analytical sample containing a buffer solution was introduced to the electrode card. Two measurements of the partial pressure of CO₂ in the solution were taken with each of the four electrodes, and the results for each electrode were averaged to yield values of 67.9, 68.0, 68.0, and 68.2 mm Hg. At time t, the analytical sample was changed to a solution containing the same buffer solution plus 10 mg/dL thiopental, and the four electrodes returned CO₂ partial pressure values of 70.4, 68.8, 68.8, and 73.4 mm Hg. The PVC-COOH based CO₂ electrodes display negligible potential drifts after analyzing a sample contaminated with thiopental as compared to the drift displayed by the HMW-PVC based CO₂ electrode, which illustrates the efficacy of PVC-COOH in preventing interference with CO₂ measurements caused by lipophilic anionic contaminants.

### Example 6

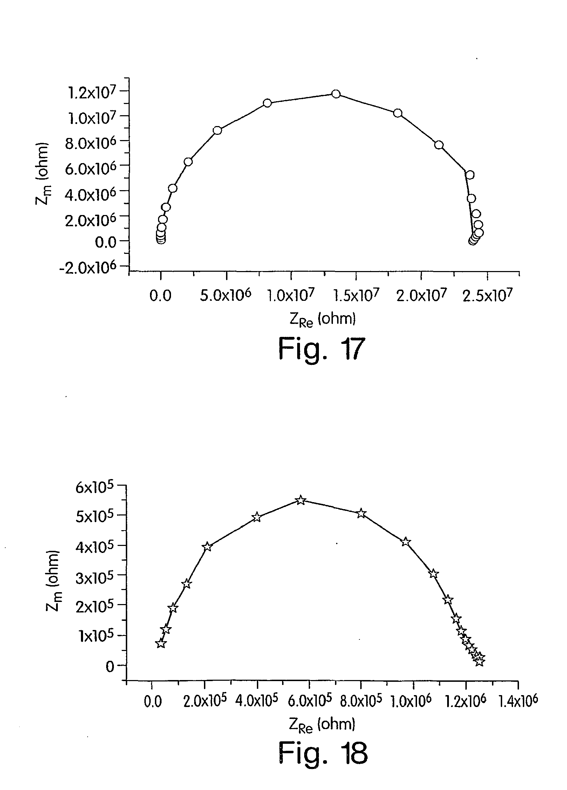

Figure 17 is a graphical representation of the bulk membrane resistance of an ISE polymeric membrane that includes HMW-PVC as a polymer component, as is known in the art. According to Figure 17, the HMW-PVC membrane has a bulk resistance of about 2.4 x 10⁷ ohms. By comparison, Figure 18 shows that the bulk membrane resistance of an ISE polymeric membrane that includes PVC-COOH as a polymer component according to the invention is about 1.25 x 10⁶ ohms, which is over nineteen times lower than that of an ISE containing HMW-PVC. By lowering the bulk membrane resistance, an ISE fabricated using PVC-COOH in its polymeric membrane exhibits enhanced potential stability and reproducibility of measurements.

### Example 7

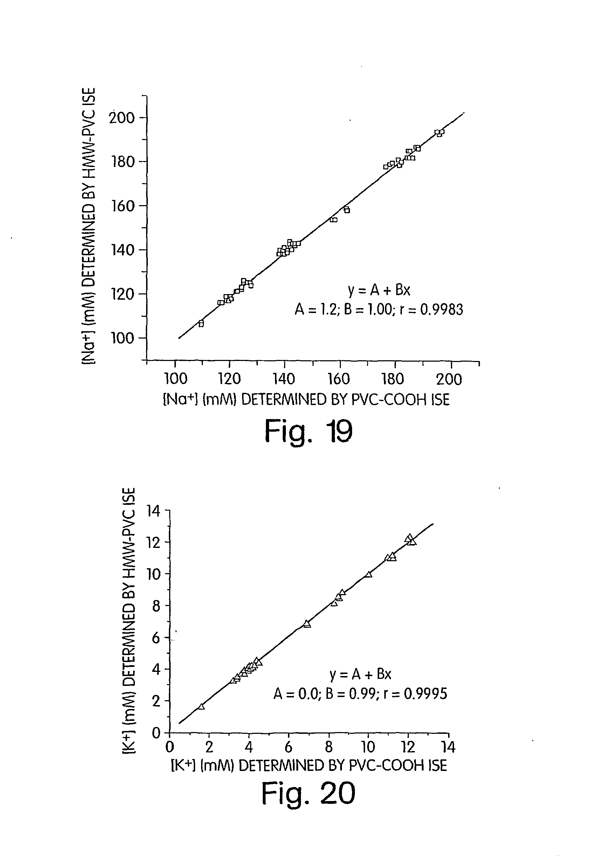

Figure 19 is a graphical representation comparing sodium concentration values in whole blood samples determined by a known sodium ISE with a polymeric membrane containing HMW-PVC against those determined by an electrode card including five sodium ISEs with polymeric membranes containing PVC-COOH according to the invention. Sodium concentration values for ninety-nine different whole blood samples representing a wide range of sodium levels were first determined with a HMW-PVC based sodium ISE, then with a PVC-COOH based sodium ISE. As illustrated by Figure 19, the values obtained from the PVC-COOH based sodium ISE correlate well with those obtained using the HMW-PVC based sodium ISE (r = 0.9983), which indicates the PVC-COOH based sodium ISE measures sodium with equal precision and accuracy as a known sodium electrode.

Figure 20 illustrates a similar experiment using a PVC-COOH based potassium ISE. Potassium concentration values for 127 different whole blood samples representing a wide range of potassium levels were first determined using a HMW-PVC based potassium ISE, then a PVC-COOH based potassium ISE. As illustrated by Figure 20, the values obtained from the PVC-COOH based potassium ISE correlate well with those obtained using the HMW-PVC based electrode (r = 0.9995), which indicates that the PVC-COOH based potassium ISE measures potassium with equal precision and accuracy as a known potassium electrode.

### Example 8

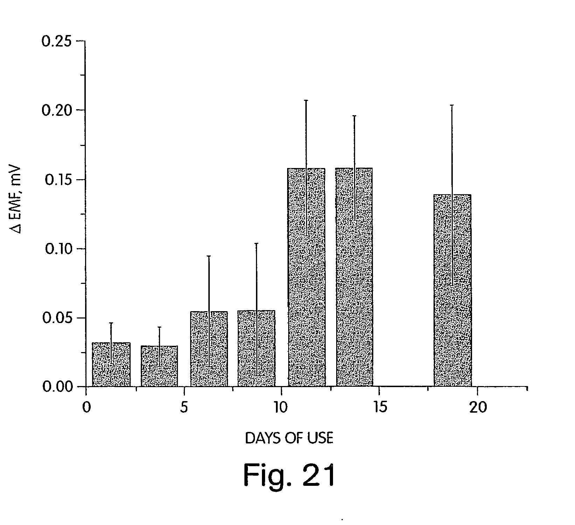

Figure 21 is a graphical representation of the stability over time of a PVC-COOH based sodium ISE according to the invention. On day 1, an analytical sample containing a known concentration of sodium was introduced to an electrode card including five sodium ISEs with polymeric membranes containing PVC-COOH according to the invention, and the concentration of sodium was measured. Then the analytical sample was changed to a solution containing the same concentration of sodium plus 10 mg/dL thiopental, and the concentration of sodium was measured again. The difference between the two measurements (ΔEMF), which represents the drift in the electrode's potential due to thiopental interference, was calculated, and the analytical sample was removed from the sensor card. This procedure was repeated on days 2, 4, 6, 9, 12, 14, and 20, and the ΔEMF values were calculated. As Figure 21 illustrates, the effect that a lipophilic anionic species such as thiopental has on a PVC-COOH based sodium ISE according to the invention remains very low throughout the life of the electrode.

## Claims

1. An in vitro diagnostic method, comprising:
selecting patients treated with lipophilic anionic agents selected from the group consisting of an analgesic, an anesthetic, thiopental sodium, phenytoin, ibuprofen, salicylate, valproate and ε-amino-caproate;
providing a sensor that reduces interference caused by lipophilic anionic agents comprising an electrode and a carboxylated polyvinyl chloride polymeric membrane;
contacting a body fluid from said patient with said sensor;
measuring a target analyte in said patient body fluid; and,
determining a diagnosis from the measurement of said target analyte.

2. The method according to claim 1, wherein the analyte comprises an inorganic cation.

3. The method according to claim 2, wherein the inorganic cation comprises: sodium, potassium, calcium, hydrogen, lithium, ammonium, or magnesium.

4. The method according to claim 1, wherein the target analyte comprises a dissolved gas; and optionally wherein the dissolved gas comprises carbon dioxide.

5. The method according to claim 1, wherein the target analyte comprises a biological metabolite.

6. The method according to claim 5, wherein either the biological metabolite comprises urea, or the biological metabolite comprises a member of the group consisting of carbohydrates, peptides, lipids, and nucleotides.

7. The method according to claim 1, wherein the carboxylated polyvinyl chloride contains 0.1 to 5 % carboxyl groups by weight.

8. The method according to claim 1, wherein the biological sample comprises blood or urine.

## Patentansprüche

1. In-vitro-Diagnoseverfahren, mit den Schritten:
Auswählen von Patienten, die mit lipophilen anionischen Mitteln behandelt werden, die aus der aus einem Analgetikum, Anästhetikum, Thiopentalnatrium, Phenytoin, Ibuprofen, Salicylat, Valproat und ε-Amino-Kaproat bestehenden Gruppe ausgewählt sind;
Bereitstellen eines Sensors, der durch lipophile anionische Mittel bewirkte Überlagerungen reduziert, umfassend eine Elektrode und eine carboxylierte Polyvinylchlorid-Polymermembran;
Inkontaktbringen eines Körperfluids aus dem Patienten mit dem Sensor;
Messen eines Zielanalyten in dem Körperfluid des Patienten; und
Erstellen einer Diagnose aus der Messung des Zielanalyten.

2. Verfahren nach Anspruch 1, wobei der Analyt ein anorganisches Kation aufweist.

3. Verfahren nach Anspruch 2, wobei das anorganische Kation aufweist: Natrium, Kalium, Calzium, Wasserstoff, Lithium, Ammonium oder Magnesium.

4. Verfahren nach Anspruch 1, wobei der Zielanalyt ein gelöstes Gas aufweist; und wobei fakultativ das gelöste Gas Kohlendioxid aufweist.

5. Verfahren nach Anspruch 1, wobei der Zielanalyt ein biologisches Stoffwechselprodukt aufweist.

6. Verfahren nach Anspruch 5, wobei entweder das biologische Stoffwechselprodukt Harnstoff aufweist oder das biologische Stoffwechselprodukt ein Mitglied der aus Kohlenhydraten, Peptiden, Lipiden und Nukleotiden bestehenden Gruppe aufweist.

7. Verfahren nach Anspruch 1, wobei das carboxylierte Polyvinylchlorid 0,1 bis 5 Gewichtsprozent Carboxylgruppen enthält.

8. Verfahren nach Anspruch 1, wobei die biologische Probe Blut oder Urin aufweist.

## Revendications

1. Procédé de diagnostic *in vitro,* comprenant :
la sélection de patients traités avec des agents anioniques lipophiles choisis dans le groupe constitué par un analgésique, un anesthésique, le thiopental sodique, la phénytoïne, l'ibuprofène, le salicylate, le valproate et 1'ε-aminocaproate ;
la mise à disposition d'un capteur qui réduit l'interférence provoquée par les agents anioniques lipophiles, comprenant une électrode et une membrane polymère à base de polychlorure de vinyle carboxylé ;
la mise en contact d'un fluide corporel dudit patient avec ledit capteur ;
la mesure d'un analyte cible dans ledit fluide corporel du patient ; et,
la formation d'un diagnostic à partir de la mesure dudit analyte cible.

2. Procédé selon la revendication 1, dans lequel l'analyte comprend un cation inorganique.

3. Procédé selon la revendication 2, dans lequel le cation inorganique comprend le sodium, le potassium, le calcium, l'hydrogène, le lithium, l'ammonium ou le magnésium.

4. Procédé selon la revendication 1, dans lequel l'analyte cible comprend un gaz dissous et, facultativement, dans lequel le gaz dissous comprend du dioxyde de carbone.

5. Procédé selon la revendication 1, dans lequel l'analyte cible comprend un métabolite biologique.

6. Procédé selon la revendication 5, dans lequel le métabolite biologique comprend soit de l'urée, soit un membre du groupe constitué par des hydrates de carbone, des peptides, des lipides et des nucléotides.

7. Procédé selon la revendication 1, dans lequel le polychlorure de vinyle carboxylé contient de 0,1 à 5% en poids de groupes carboxyle.

8. Procédé selon la revendication 1, dans lequel l'échantillon biologique comprend du sang ou de l'urine.
